(19) Europäisches Patentamt
European Patent Office
Office européen des brevets

(11) Veröffentlichungsnummer: **0 339 390**
**A1**

(12) **EUROPÄISCHE PATENTANMELDUNG**

(21) Anmeldenummer: **89106689.6**

(22) Anmeldetag: **14.04.89**

(51) Int. Cl.[4]: **C07D 413/04 , A01N 43/76 , A01N 43/82**

(30) Priorität: **27.04.88 JP 102445/88**

(43) Veröffentlichungstag der Anmeldung: **02.11.89 Patentblatt 89/44**

(84) Benannte Vertragsstaaten: **BE CH DE FR GB IT LI NL**

(71) Anmelder: **NIHON TOKUSHU NOYAKU SEIZO K.K.**
**Itohpia Nihonbashi Honcho Building 7-1, Nihonbashi Honcho 2-chome**
**Chuo-ku Tokyo 103(JP)**

(72) Erfinder: **Kume, Toyohiko**
**6-7-8, Asahigaoka**
**Hinio-shi Tokyo(JP)**
Erfinder: **Goto, Toshio**
**3454-21, Honmachida**
**Machida-shi Tokyo(JP)**
Erfinder: **Kamochi, Atsumi**
**2-24-10, Higashi-Toyoda**
**Hino-shi Tokyo(JP)**
Erfinder: **Yanagi, Akihiko**
**2-1200-1, Nagabuchi**
**Oume-shi Tokyo(JP)**
Erfinder: **Miyauchi, Hiroshi**
**39-15, Namiki-cho**
**Hachioji-shi Tokyo(JP)**

(74) Vertreter: **Schumacher, Günter, Dr. et al**
**c/o Bayer AG Konzernverwaltung RP**
**Patentabteilung**
**D-5090 Leverkusen 1 Bayerwerk(DE)**

(54) **Herbizid aktive 2,5-Dihydropyrrole.**

(57) Offenbart werden neue 2,5-Dihydropyrrole der Formel (I),

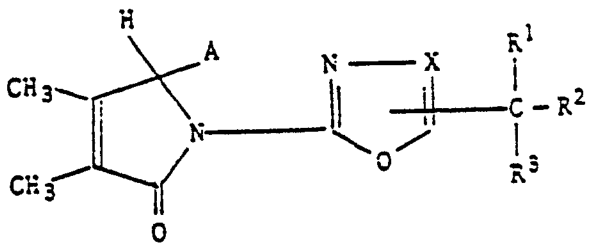

(I)

Verfahren und neue Zwischenprodukte zu ihrer Herstellung und die Verwendung der neuen Verbindungen als Herbizide.

EP 0 339 390 A1

## Herbizid aktive 2,5-Dihydropyrrole

Die vorliegende Erfindung betrifft neue 2,5-Dihydropyrrole, Verfahren und neue Zwischenprodukte für ihre Herstellung und ihre Verwendung als Herbizide.

Es wurde bereits offenbart, daß bestimmte 2,5-Dihydropyrrole eine Funktion zur Bekämpfung von Pflanzen- und Tierschädlingen aufweisen (siehe die JP-OS 23965/1978).

Nunmehr wurden neue 2,5-Dihydropyrrole der Formel (I)

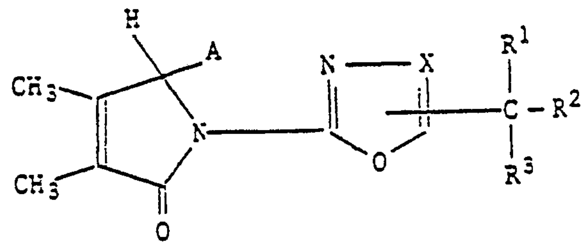

(I)

gefunden, in der

A  Hydroxy, Halogen, eine Alkylcarbonyloxy-Gruppe mit Alkyl mit 1 bis 4 Kohlenstoff-Atomen, das gegebenenfalls durch Halogen substituiert ist, oder eine Alkylsulfonyloxy-Gruppe mit 1 bis 4 Kohlenstoff-Atomen, die gegebenenfalls durch Halogen substituiert ist, bezeichnet,

X  CH oder N bezeichnet und

$R^1$,$R^2$ und $R^3$  jeweils Wasserstoff oder eine Alkyl-Gruppe mit 1 bis 4 Kohlenstoff-Atomen, die gegebenenfalls durch Halogen substituiert ist, bezeichnen, mit der Maßgabe, daß $R^1$, $R^2$ und $R^3$ nicht gleichzeitig Wasserstoff bezeichen, oder $R^2$ und $R^3$ zusammen mit dem Kohlenstoff-Atom, an das sie gebunden sind, einen alicyclischen Ring mit 3 bis 6 Kohlenstoff-Atomen bilden können.

Die Verbindungen der Formel (I) können mittels eines Verfahrens erhalten werden, bei dem

a) in dem Fall, in dem A Hydroxy bezeichnet, Verbin dungen der Formel (II)

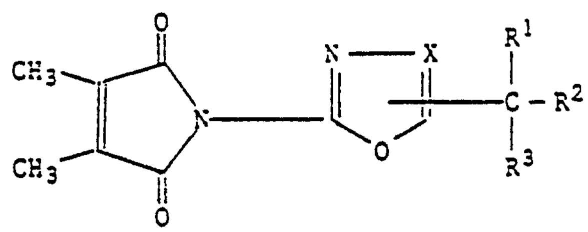

(II)

,

in der X, $R^1$, $R^2$ und $R^3$ jeweils die im Vorstehenden angegebenen Bedeutungen haben, mit einem Reduktionsmittel in Gegenwart inerter Lösungsmittel umgesetzt werden oder

b) in dem Fall, in dem A Halogen bezeichnet, Verbindungen der Formerl (Ia)

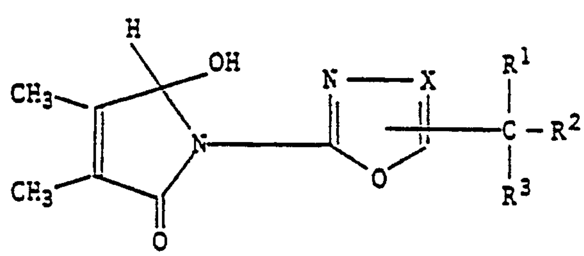

(Ia)

,

in der X, $R^1$, $R^2$ und $R^3$ jeweils die im Vorstehenden angegebenen Bedeutungen haben, mit einem Halogenierungsmittel in Gegenwart inerter Lösungsmittel umgesetzt werden oder

c) in dem Fall, in dem A eine Alkylcarbonyloxy-Gruppe mit einem Alkyl mit 1 bis 4 Kohlenstoff-Atomen, das gegebenenfalls durch Halogen substituiert ist, oder eine Alkylsulfonyloxy-Gruppe mit 1 bis 4 Kohlenstoff-Atomen, die gegebenenfalls durch Halogen substituiert ist, bezeichnet, A durch -O-W ersetzt wird,
die Verbindungen der oben bezeichneten Formerl (Ia) mit Verbindungen der Formel (III)
W - M (III),
worin
W eine Alkylcarbonyl-Gruppe mit einem Alkyl mit 1 bis 4 Kohlenstoff-Atomen, das gegebenenfalls durch Halogen substituiert ist, oder eine Alkylsulfonyl-Gruppe mit 1 bis 4 Kohlenstoff-Atomen, die gegebenenfalls durch Halogen substituiert ist, bezeichnet und
M Halogen bezeichnet, oder
mit Verbindungen der Formerl (IV)
W - O - W (IV),
worin W die im Vorstehenden angegebenen Bedeutungen hat,
in Gegenwart inerter Lösungsmittel und gegebenenfalls in Gegenwart einer Base umgesetzt werden.

Die erfindungsgemäßen 2,5-Dihydropyrrole der Formel (I) zeigen eine starke herbizide Wirkung.

Die 2,5-Dihydropyrrole der Formel (I) sind neu, auch wenn die Formel (I) generisch von dem Anspruch der oben genannten JP-OS 23965/1978 umfaßt wird.

Überraschenderweise zeigen die erfindungsgemäßen 2,5-Dihydropyrrole eine wesentlich stärkere selektive herbizide Wirkung als die aus dem oben genannten Stand der Technik, der JP-OS 23965/1978 bekannten, und sie besitzen gleichzeitig auch eine gute Verträglichkeit mit Nutzpflanzen.

Unter den erfindungsgemäßen 2,5-Dihydropyrrole der Formel (I) sind bevorzugte Verbindungen diejenigen, in denen
A Hydroxy, Chlor, Brom, eine Alkylcarbonyloxy-Gruppe mit einem Alkyl mit 1 bis 2 Kohlenstoff-Atomen, das gegebenenfalls durch Fluor und/oder durch Chlor substituiert ist, oder eine Alkylsulfonyloxy-Gruppe mit 1 bis 2 Kohlenstoff-Atomen, die gegebenenfalls durch Fluor und/oder durch Chlor substituiert ist, bezeichnet,
X CH oder N bezeichnet,
$R^1$ Methyl oder Ethyl bezeichnet und
$R^2$ und $R^3$ jeweils Methyl, Ethyl oder eine durch Chlor oder Fluor substituierte Alkyl-Gruppe mit 1 bis 2 Kohlenstoff-Atomen bezeichnen oder $R^2$ und $R^3$ zusammen mit dem Kohlenstoff-Atom, an das sie gebunden sind, Cyclopropan bilden können.

Ganz besonders bevorzugte 2,5-Dihydropyrrole der Formel (I) sind diejenigen, in denen
A Hydroxy, Chlor, Brom, Acetyloxy, Trifluoroacetyloxy oder Methylsulfonyloxy bezeichnet,
X CH oder N ist,
$R^1$ Methyl ist und
$R^2$ und $R^3$ jeweils Methyl oder Fluoromethyl sind.

Speziell seien die folgenden Verbindungen erwähnt:
N-(5-tert-Butyl-1,3-oxazol-2-yl)-3,4-dimethyl-2-hydroxy-5-oxo-2,5-dihydropyrrol;
N-(5-tert-Butyl-1,3,4-oxadiazol-2-yl)-3,4-dimethyl-2-hydroxy-5-oxo-2,5-dihydropyrrol; und
N-(5-tert-Butyl-1,3,4-oxadiazol-2-yl)-2-chloro-3,4-dimethyl-5-oxo-2,5-dihydropyrrol.

Wenn als Ausgangsstoffe in dem Verfahren (a) beispielsweise N-(5-tert-Butyl-1,3-oxazol-2-yl)-2,3-dimethylmaleinimid und Natriumborhydrid eingesetzt werden, kann die Reaktion durch das folgende Formelschema ausgedrückt werden:

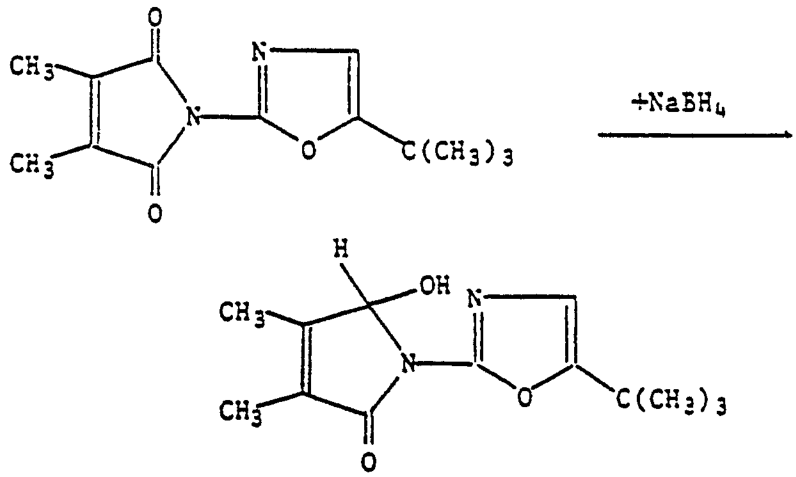


Wenn als Ausgangsstoffe in dem Verfahren (b) beispielsweise N-(5-tert-Butyl-1,3-oxazol-2-yl)-3,4-dimethyl-2-hydroxy-5-oxo-2,5-dihydropyrrol und Thionylchlorid eingesetzt werden, kann die Reaktion durch das folgende Formelschema ausgedrückt werden:

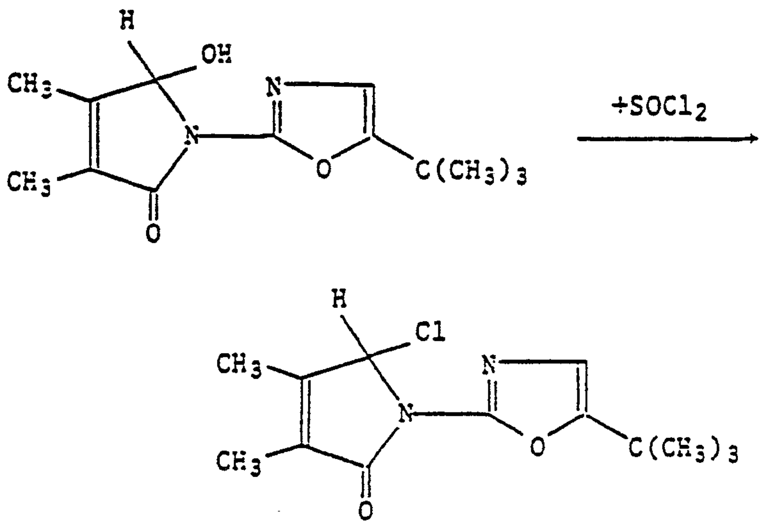


Wenn als Ausgangstoffe in dem Verfahren (c) beispielsweise N-(5-tert-Butyl-1,3-oxazol-2-yl)-3,4-dimethyl-2-hydroxy-5-oxo-2,4-dihydropyrrol und Acetylchlorid eingesetzt werden, kann die Reaktion durch das folgende Formelschema ausgedrückt werden:

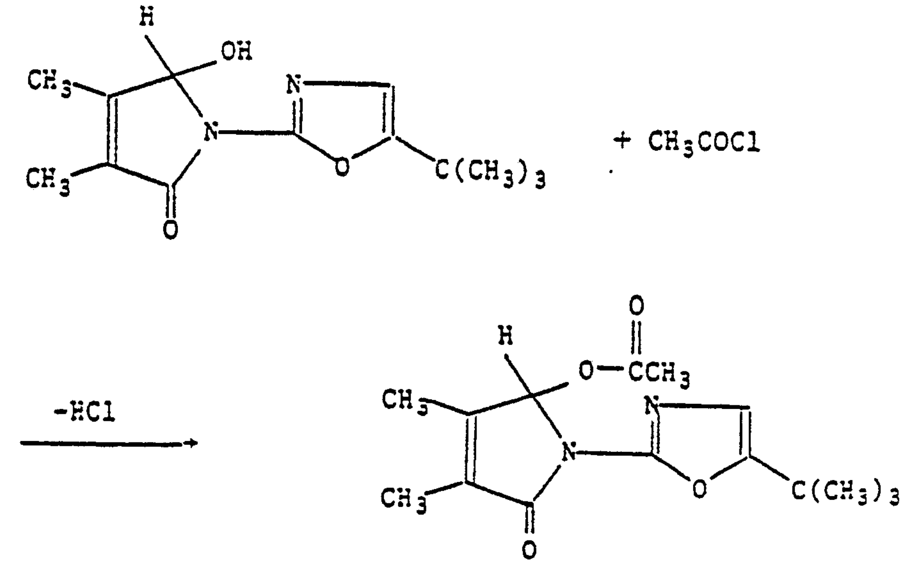


In dem Verfahren (a) haben X, $R^1$, $R^2$ und $R^3$ in der Formel (II) der Ausgangstoffe jeweils die gleichen Bedeutungen, wie sie im Vorstehenden angegeben sind.

Vorzugsweise haben X, $R^1$, $R^2$ und $R^3$ in der Formel (II) jeweils die gleichen bevorzugten Bedeutungen, wie sie oben angegeben sind.

Die Verbindungen der Formel (II) sind neu und können dadurch hergestellt werden, daß eine Verbindung der Formel

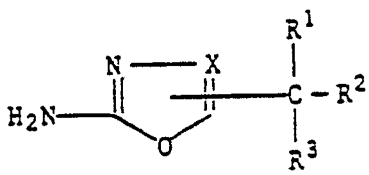
(V)

,

in der X, $R^1$, $R^2$ und $R^3$ jeweils die gleichen Bedeutungen wie im Vorstehenden haben, entweder mit 2,3-Dimethylmaleinsäureanhydrid oder mit 2 mol Maleinsäureanhydrid auf 1 mol der Verbindung der Formel (V) umgesetzt werden.

Von den zwei oben genannten Verfahren zur Herstellung der Verbindungen der Formel (II) kann das erstgenannte Verfahren in der in der JP-OS 23965/1978 offenbarten Weise durchgeführt werden, und das zweite Verfahren kann in der in der JP-OS 46209/1977 aufgezeigten Weise durchgeführt werden.

Die 2-Aminooxazole, in denen die Gruppe

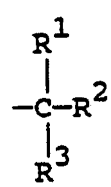

in der Formel (V) in der 4- oder 5-Stellung gebunden ist, können nach einem Verfahren hergestellt werden, das in Ann., Band 467, S. 262, und in Japan. Pharmaceutical Journal, Band 87, Nr. 1, S. 14 (1967) und ibid., Band 91, Nr. 4, S. 425-435 (1971), beschrieben ist.

Die 2-Aminooxazole, in denen die Gruppe

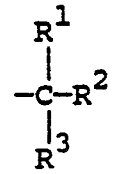

in der Formel (V) in der 5-Stellung gebunden ist, können nach einem Verfahren hergestellt werden, das in der US-PS 2 883 391, der DD-PS 52 668 oder Khim. Geterotsiki Soedin, Band 11, S. 1553-1556 (1986), beschrieben ist.

Alternativ kann 2-Amino-5-tert-butyl-1,3-oxazol, zusammen mit 2-Amino-4-tert-butyl-1,3-oxazol, durch Umsetzung von tert-Butyl-chloromethyl-keton mit Cyanamid erhalten werden.

In dem vorstehenden Verfahren kann 2-Amino-4-tert-butyl-1,3-oxazol auch nach einer in Aust. J. Chem., Band 38, S. 447 (1985) beschriebenen Methode erhalten werden.

Beispiele für Verbindungen der Formel (V) sind 2-Amino-4-tert-butyl-1,3-oxazol, 2-Amino-5-tert-butyl-1,3-oxazol und dergleichen.

Beispiele für Reduktionsmittel, die in dem Verfahren (a) eingesetzt werden können, sind Metallhydride wie Natriumborhydrid, Lithiumaluminiumhydrid und dergleichen.

Beispiele für Halogenierungsmittel, die in dem Verfahren (b) eingesetzt werden können, sind Thionylchlorid und dergleichen.

In den Ausgangs-Verbindungen der Formeln (III) und (IV), die in dem Verfahren (c) eingesetzt werden, haben die Symbole W und M in den Formeln die oben angegebenen Bedeutungen. Vorzugsweise bezeichnet W eine Alkylcarbonyl-Gruppe mit 1 oder 2 Kohlenstoff-Atomen, die gegebenenfalls durch Fluor und/oder durch Chlor substituiert ist, oder eine Alkylsulfonyloxy-Gruppe mit 1 bis 2 Kohlenstoff-Atomen, die gegebenenfalls durch Fluor und/oder durch Chlor substituiert ist, und M bezeichnet Chlor.

Zu den Verbindungen der Formeln (III) und (IV) zählen bekannte Verbindungen wie Acetylchlorid, Methansulfonylchlorid, Trifluoroessigsäureanhydrid, Essigsäureanhydrid etc..

Als geeignete Verdünnungsmittel für die Durchführung des Verfahrens (a) können alle Arten inerter organischer Lösungsmittel erwähnt werden.

Beispiele für diese Verdünnungsmittel sind Ether wie Dioxan und Tetrahydrofuran; Alkohole wie Methanol, Ethanol, Isopropanol, Butanol und Ethylenglycol; Säureamide wie N,N-Dimethylformamid und dergleichen.

Die Reaktionstemperatur des Verfahrens (a) kann innerhalb eines Bereichs von beträchtlicher Breite variiert werden. Im allgemeinen wird die Reaktion bei einer Temperatur von etwa -20 °C bis +80 °C, vorzugsweise bei einer Temperatur von etwa 0 °C bis 30 °C durchgeführt.

Es wird bevorzugt, die Reaktion unter normalen Druck durchzuführen, wenngleich auch ein höherer oder niedrigerer Druck angewandt werden kann.

Bei der Durchführung des Verfahrens (a) ist es möglich, die Verbindungen der Formerl (II) einer reduzierenden Reaktion mit Hilfe eines oben genannten Reduktionsmittels zu unterwerfen, so daß die angestrebten Verbindungen der Formel (I) erhalten werden können, wie in den nachstehend angegebenen Beispielen gezeigt wird.

Bei der Durchführung des Verfahrens (b) können geeignete Verdünnungsmittel verwendet werden, darunter Kohlenwasserstoffe wie Benzol, Toluol und Xylol; halogenierte Kohlenwasserstoffe wie Methylenchlorid, Chloroform, Kohlenstofftetrachlorid, Chlorobenzol etc..

Das Verfahren (b) kann im wesentlichen bei Temperaturen innerhalb eines breiten Bereichs durchgeführt werden. Beispielsweise kann es bei einer Temperatur von etwa -20 °C bis 150 °C, vorzugsweise von etwa 0 °C bis 80 °C, durchgeführt werden.

Die Reaktion wird vorzugsweise unter normalen Druck durchgeführt, jedoch kann auch bei einem höheren oder niedrigeren Druck gearbeitet werden.

Bei der Durchführung des Verfahrens (b) ist es möglich, die Verbindungen der Formel (Ia) einer Chlorierungsreaktion mit Hilfe von Thionylchlorid zu unterwerfen, so daß die angestrebten Verbindungen der Formel (I) leicht erhalten werden können.

Bei der Durchführung des Verfahrens c) können geeignete Verdünnungsmittel verwendet werden, beispielsweise Ether wie Dioxan und Tetrahydrofuran; Säureamide wie N,N-Dimethylformamid, N,N-Dimethylacetamid und Hexamethylphosphorsäuretriamid, Sulfoxide wie Dimethylsulfoxid und dergleichen.

Das Verfahren (c) kann in Gegenwart eines säurebindenden Mittels durchgeführt werden. Beispiele für die säurebindenden Mittel sind Alkalimetallhydride; tertiäre Amine wie Triethylamin, Diethylanilin, Pyridin, 4-Dimethylaminopyridin, 1,8-Diazabicyclo[5,4,0]undec-7-en (DBU), 1,4-Dizabicyclo[2,2,2]octan (DABCO) und

dergleichen.

Das Verfahren kann (c) im wesentlichen bei Temperaturen innerhalb eines breiten Bereichs durchgeführt werden. Es kann beispielsweise bei einer Temperatur innerhalb von etwa 50 °C bis 200 °C, vorzugsweise innerhalb von etwa 80 °C bis 160 °C durchgeführt werden.

Die Reaktion wird vorzugsweise unter normalen Druck durchgefährt, jedoch kann auch unter einem höheren oder niedrigeren Druck gearbeitet werden.

Bei der Durchführung des Verfahrens (c) können etwa 1 bis 1,5 mol der Verbindungen der Formel (III) oder (IV) pro 1 mol der Verbindungen der Formel (Ia) eingesetzt werden. Diese Verbindungen können miteinander in einem inerten Lösungsmittel, gegebenenfalls in Gegenwart eines säurebindenden Mittels, umgesetzt werden, so daß die angestrebten Verbindungen der Formel (I) erhalten werden können.

Die erfindunsgemäßen Verbindungen der Formel (I) haben ein asymmetrisches Kohlenstoff-Atom in der 2-Position des 2,5-Dihydropyrrol-Rings. Demgemäß schließen die 2,5-Dihydropyrrole der Formel (I) gemäß der Erfindung die optischen Isomeren ein.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht wind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendt werden:

Dikotyledonen-Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von der Konzentration eigen sich die Verbindungen zur totalen Bekämpfung von Unkräutern, beispielsweise auf Industriegelände und Bahnkörpern sowie auf Wegen und Plätzen mit oder ohne Baumbestand. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Präparate wie Lösungen, Emulsionen, Suspensionen, Pulver, Schäume, Pasten, Granulat, Aerosole, mit dem Wirkstoff imprägnierte natürliche und synthetische Materialien,

sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dis pergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalen Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Fabstoffe oder Metallphthalocyanin-Farbstoffe, und Spurennährstoffe wie Salze von Eisen, Mangan, Bor, Kupfer, Cobalt, Molydän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate auch zur Unkrautbekämpfung als Mischungen mit bekannten Herbiziden eingesetzt werden, wobei Fertigpräparate oder Tankmischungen möglich sind.

Mischungen mit anderen bekannten aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die eingesetzten Mengen der aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs zwischen etwa 0.0005 und etwa 3 kg/ha, vorzugsweise zwischen etwa 0,001 und etwa 2 kg/ha.

Die Herstellung und Verwendung der erfindungsgemäßen Verbindungen sind den folgenden Beispielen zu entnehmen.

Herstellungsbeispiele

Beispiel 1

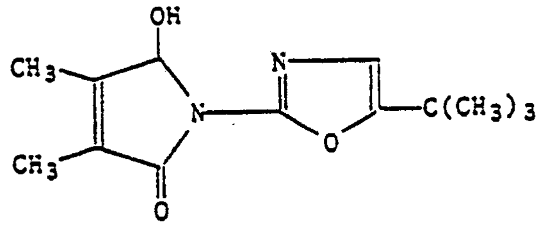

Eine Mischung von 2-Amino-5-tert-butyl-1,3-oxazol (1,40 g) und 2,3-Dimethylmaleinsäureanhydrid (1,51 g) in Toluol (30 ml) wurde 2 h unter Rückfluß erhitzt. Das während der Reaktion gebildete Wasser wurde mit Hilfe einer Dean-Stark-Vorrichtung entfernt. Dann wurde Essigsäure (5 ml) zugegeben, und die Reaktionsmischung wurde weiter 2 h erhitzt. Nach Beendigung der Reaktion wurden das Lösungsmittel und das überschüssige Säureanhydrid unter vermindertem Druck entfernt. Der Rückstand wurde in Methanol (50 ml) gelöst, und Natriumborhydrid (0,38 g) wurde portionsweise bei Raumtempertur hinzugefügt. Danach wurde die Reaktionsmischung 3 h bei Raumtemperatur gerührt. Nach dieser Reaktion wurde Essigsäure (0,5 ml) zugegeben, und das Lösungsmittel wurde unter vermindertem Druck abdestilliert. Der Rückstand wurde mit Wasser vermischt, mit Kaliumcarbonat alkalisch gemacht und mit Methylenchlorid (2 x 50 ml) extrahiert. Nach dem Trocknen der Reaktionsmischung über wasserfreiem Magnesiumsulfat wurde das Lösungsmittel unter vermindertem Druck abdestilliert, und der Rückstand wurde aus Hexan umkristallisiert, wonach die gewünschte Verbindung, d.h. N-(5-tert-Butyl-1,3-oxazol-2-yl)-3,4-dimethyl-2-hydroxy-5-oxo-2,5-dihydropyrrol (2,15 g) erhalten wurde; Schmp. 103-104 °C.

Beispiel 2

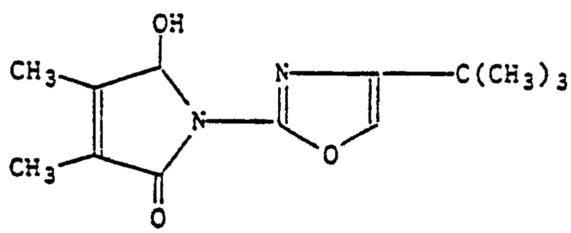

In diesem Fall wurden 2-Amino-4-tert-butyl-1,3-oxazol (1,40 g), 2,3-Dimethylmaleinsäureanhydrid (1,51 g) und Natriumborhydrid (0,38 g) zur Durchführung der Reaktion in ähnlicher Weise wie in Beispiel 1 eingesetzt. Das resultierende Produkt wurde aus einem Toluol/Hexan-Gemisch umkristallisiert, wonach die gewünschte Verbin dung, d.h. N-(4-tert-Butyl-1,3-oxazol-2-yl)-3,4-dimethyl-2-hydroxy-5-oxo-2,5-dihydropyrrol (2,10 g) erhalten wurde; Schmp. 171-174 °C.

In der gleichen Weise, wie sie in den vorstehenden Beispielen aufgezeigt wurde, kann eine Anzahl von Verbindungen gemäß der Erfindung hergestellt werden. Diese Verbindungen sowie die in den Beispielen 1 und 2 hergestellten Verbindungen sind in Tabelle 1 aufgeführt.

## Tabelle 1

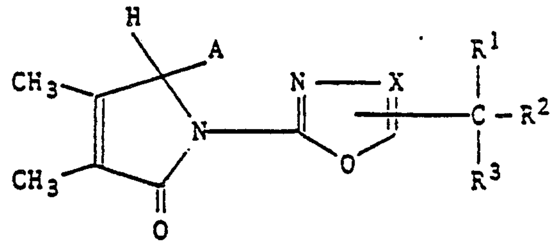

| Verbindung Nr. | A | 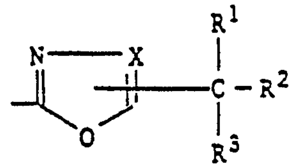 | Physikalische Konstante |
|---|---|---|---|
| 1 | -OH | 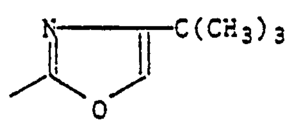 | Schmp. 171 - 174°C |
| 2 | $-OCOCH_3$ | 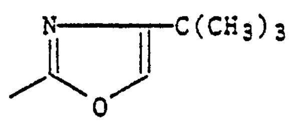 | $n_D^{50}$ 1.4955 |
| 3 | $-OCOCF_3$ | 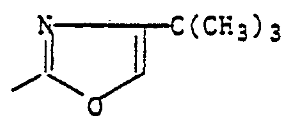 | $n_D^{50}$ 1.485 |
| 4 | $-OSO_2CH_3$ | 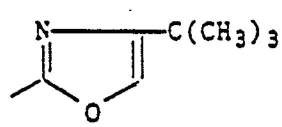 | $n_D^{20}$ 1.4980 |
| 5 | -Cl | 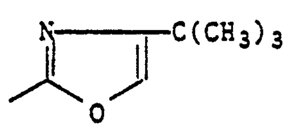 | Viskoses Öl |
| 6 | -OH | 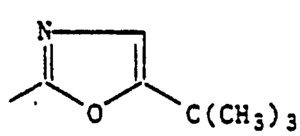 | Schmp.103 - 104°C |

## Tabelle 1 - Fortsetzung

| Verbindung Nr. | A | 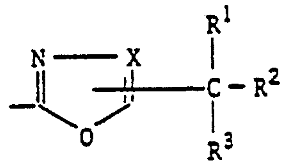 | Physikalische Konstante |
|---|---|---|---|
| 7 | $-OCOCH_3$ | 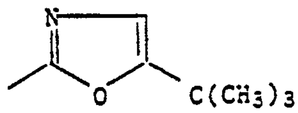 | Schmp. 96 - 101°C |
| 8 | -Cl | 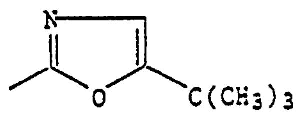 | Viskoses Öl |
| 9 | -OH | 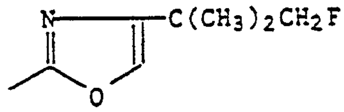 | |
| 10 | -OH | 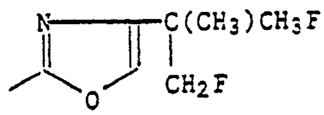 | |
| 11 | -OH | 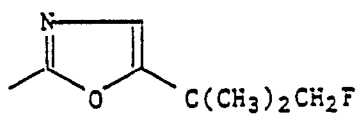 | |
| 12 | -OH | 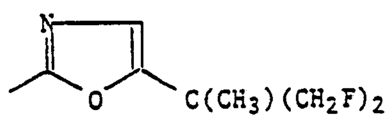 | |
| 13 | -OH | 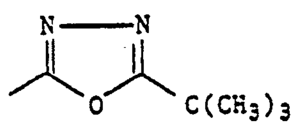 | Schmp. 142 - 144°C |
| 14 | $-OCOCH_3$ | 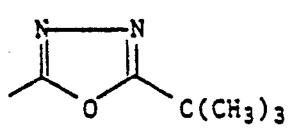 | $n_D^{50}$ 1.4935 |

## Tabelle 1 - Fortsetzung

| Verbindung Nr. | A | 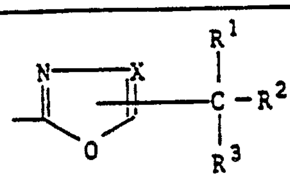 | Physikalische Konstante |
|---|---|---|---|
| 15 | $-OCOCF_3$ | 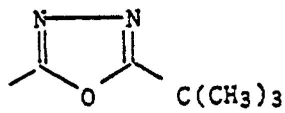 | $n_D^{50}$ 1.485 |
| 16 | $-OSO_2CH_3$ | 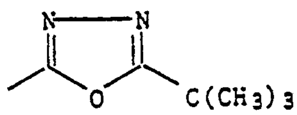 | $n_D^{50}$ 1.5025 |
| 17 | -Cl | 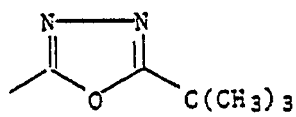 | Schmp. 112 - 116°C |
| 18 | -Br | 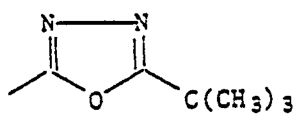 | Schmp. 94 - 98°C |
| 19 | -OH | 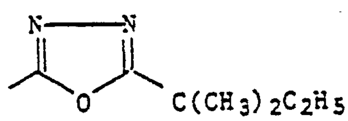 | |
| 20 | -OH | 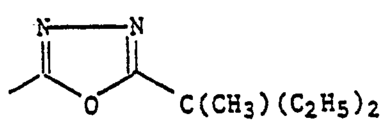 | |
| 21 | -OH | 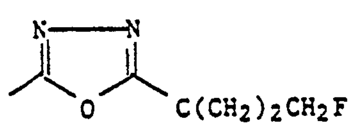 | |
| 22 | -OH | 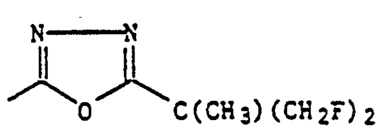 | |

Tabelle 1 - Fortsetzung

| Verbindung Nr. | A | 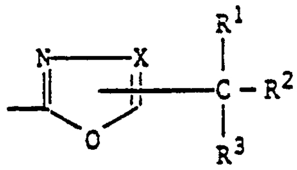 | Physikalische Konstante |
|---|---|---|---|
| 23 | -OH | 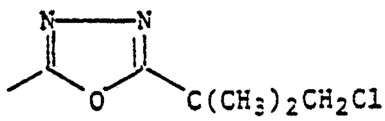 | |
| 24 | -OH | 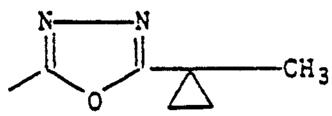 | |

Biologische Test-Beispiele

Zum Vergleich eingesetzte bekannte Verbindung:

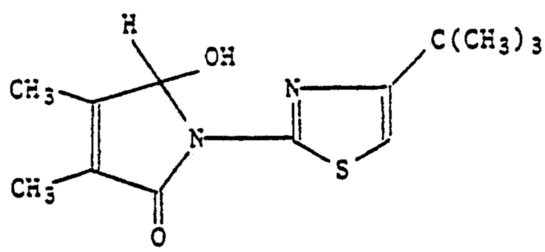

(E-1)

(offenbart in der JP-OS 23965/1978).

Beispiel 3

Test gegen Unkräuter auf höher gelegenem Ackerboden durch Bodenbehandlung vor dem Auflaufen:

| Herstellung eines Wirkstoff-Präparats | |
|---|---|
| Träger: | 5 Gewichtsteile Aceton; |
| Emulgator: | 1 Gewichtsteil Benzyloxypolyglycolether. |

Ein Wirkstoff-Präparat in Form eines emulgierbaren Konzentrats wurde erhalten durch Vermischen von

1 Gewichtsteil der aktiven Verbindung mit den oben angegebenen Mengen des Trägers und des emulgierenden Mittels. Eine vorher festgelegte Menge des Präparats wurde mit Wasser verdünnt.

Test-Verfahren

In einem Gewächshaus wurden Mais-Samen in Töpfe von 500 $cm^2$ Fläche, die mit Ackererde gefüllt waren, eingesät, und Erde, die Samen von Hühnerhirse (Echinochloa crus-galli), Digitaria (Digitaria sanguinalis), grauem Amaranth (Amaranthus lividus L.) und Gänsefuß (Chenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration gleichmäßig über die Oberflächenschicht der Erde in jedem der Test-Töpfe gesprüht.

Vier Wochen nach dem Sprühen wurden die herbizide Wirkung und die Phytotoxizität gegenüber den Nutzpflanzen untersucht und mit Hilfe der folgendent Skala von 0 bis 5 bewertet:

| Bewertung Herbizide Wirkung (bewertet als Unkrautvernichtungs-Verhältnis, bezogen auf eine unbehandelte Fläche) | |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |
| Bewertung Phytotoxizität gegen Nutzpflanzen (bewertet unter Bezug auf die unbehandelte Fläche) | |
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Test-Ergebnisse sind in Tabelle 2 aufgeführt.

Tabelle 2

| Aktive Verbindung Nr. | Wirkstoff-Menge kg/ha | Herbizide Wirkung gegen Unkräuter | | | | Phytotoxizität gegen Mais-Pflanzen |
|---|---|---|---|---|---|---|
| | | Hühnerhirse | Digitaria | grauer Amaranth | Gänsefuß | |
| 6 | 0,5 | 5 | 5 | 5 | 5 | 1 |
| | 0,25 | 4 | 5 | 5 | 5 | 0 |
| 7 | 0,5 | 5 | 5 | 5 | 5 | 1 |
| | 0,25 | 4 | 5 | 5 | 5 | 0 |
| 13 | 0,5 | 4 | 5 | 5 | 5 | 0 |
| | 0,25 | 4 | 4 | 5 | 5 | 0 |
| Bekannte Verbindung | | | | | | |
| E-1 | 0,5 | 1 | 1 | 2 | 1 | 0 |
| | 0,25 | 0 | 0 | 0 | 0 | 0 |

Beispiel 4

Test gegen Unkräuter auf höher gelegenem Ackerboden durch Laub-Behandlung:

In einem Gewächshaus wurden Mais-Samen in Töpfe von 500 $cm^2$ Fläche, die mit Ackererde gefüllt waren, eingesät, und Erde, die Samen von Digitaria (Digitaria sanguinalis), grauem Amaranth (Amaranthus lividus L.), Gänsefuß (Chenopodium album L.) und Portulak (Portulaca oleracea L.) enthielt, wurde über die Erde in den Töpfen in einer Tiefe von 1 cm gestreut.

Nach der Einsaat wurden die Pflanzen 14 Tage angezogen, und eine Test-Chemikalie in einer vorher festgelegten Konzentration, hergestellt wie in Beispiel 3, wurde gleichmäßig über die Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 3 geprüft. Die Ergebnisse sind in Tabelle 3 aufgeführt.

Tabelle 3

| Aktive Verbindung Nr. | Wirkstoff-Menge kg/ha | Herbizide Wirkung gegen Unkräuter | | | | Phytotoxizität gegen Mais-Pflanzen |
|---|---|---|---|---|---|---|
| | | Digitaria | grauer Amaranth | Gänsefuß | Portulak | |
| 6 | 0,5 | 5 | 5 | 5 | 5 | 0 |
| | 0,25 | 5 | 5 | 5 | 5 | 0 |
| 13 | 0,5 | 5 | 5 | 5 | 5 | 0 |
| | 0,25 | 5 | 5 | 5 | 5 | 0 |
| 17 | 0,5 | 5 | 5 | 5 | 5 | 1 |
| | 0,25 | 5 | 5 | 5 | 5 | 0 |
| Bekannte Verbindung | | | | | | |
| E-1 | 0,5 | 1 | 2 | 2 | 1 | 0 |
| | 0,25 | 0 | 1 | 1 | 0 | 0 |

**Ansprüche**

1. 2,5-Dihydropyrrole der Formel (I)

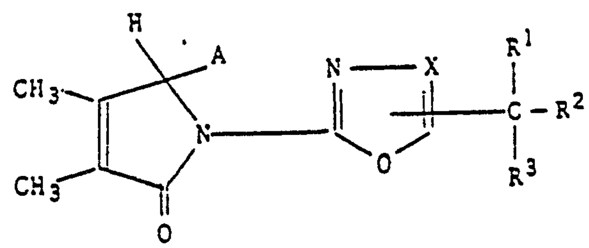

(I)

,

in der

A Hydroxy, Halogen, eine Alkylcarbonyloxy-Gruppe mit Alkyl mit 1 bis 4 Kohlenstoff-Atomen, das gegebenenfalls durch Halogen substituiert ist, oder eine Alkylsulfonyloxy-Gruppe mit 1 bis 4 Kohlenstoff-Atomen, die gegebenenfalls durch Halogen substituiert ist, bezeichnet,

X CH oder N bezeichnet und

$R^1$, $R^2$ und $R^3$ jeweils Wasserstoff oder eine Alkyl-Gruppe mit 1 bis 4 Kohlenstoff-Atomen, die

gegebenenfalls durch Halogen substituiert ist, bezeichnen, mit der Maßgabe, daß $R^1$, $R^2$ und $R^3$ nicht gleichzeitig Wasserstoff bezeichnen; oder $R^2$ und $R^3$ zusammen mit dem Kohlenstoff-Atom, an das sie gebunden sind, einen alicyclischen Ring mit 3 bis 6 Kohlenstoff-Atomen bilden können.

2. Verbindungen der Formel (I) nach Anspruch 1, worin

A Hydroxy, Chlor, Brom, eine Alkylcarbonyloxy-Gruppe mit einem Alkyl mit 1 bis 2 Kohlenstoff-Atomen, das gegebenenfalls durch Fluor und/oder durch Chlor substituiert ist, oder eine Alkylsulfonyloxy-Gruppe mit 1 bis 2 Kohlenstoff-Atomen, die gegebenenfalls durch Fluor und/oder durch Chlor substituiert ist, bezeichnet,

X CH oder N bezeichnet,

$R^1$ Methyl oder Ethyl bezeichnet und

$R^2$ und $R^3$ jeweils Methyl, Ethyl oder eine durch Chlor oder Fluor substituierte Alkyl-Gruppe mit 1 bis 2 Kohlenstoff-Atomen bezeichnen oder $R^2$ und $R^3$ zusammen mit dem Kohlenstoff-Atom, an das sie gebunden sind, Cyclopropan bilden können.

3. Verbindungen der Formel (I) nach Anspruch 1, worin

A Hydroxy, Chlor, Brom, Acetyloxy, Trifluoroacetyloxy oder Methylsulfonyloxy bezeichnet,

X CH oder N ist, $R^1$ Methyl ist und

$R^2$ und $R^3$ jeweils Methyl oder Fluoromethyl sind.

4. Verbindung nach den Ansprüchen 1 bis 3, nämlich N-(5-tert-Butyl-1,3-oxazol-2-yl)-3,4-dimethyl-2-hydroxy-5-oxo-2,5-dihydropyrrol der Formel

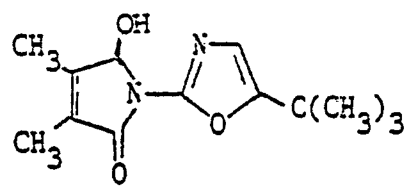

,

N-(5-tert-Butyl-1,3,4-oxadiazol-2-yl)-3,4-dimethyl-2-hydroxy-5-oxo-2,5-dihydropyrrol der Formel

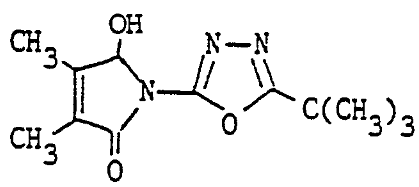

und

N-(5-tert-Butyl-1,3,4-oxadiazol-2-yl)-2-chloro-3,4-dimethyl-5-oxo-2,5-dihydropyrrol der Formel

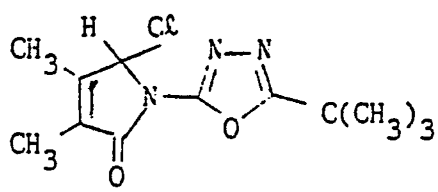

.

5. Verfahren zur Herstellung von 2,5-Dihydropyrrolen der Formel (I)

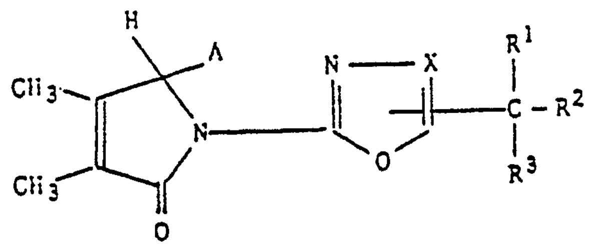

(I)

,

in der

A Hydroxy, Halogen, eine Alkylcarbonyloxy-Gruppe mit einem Alkyl mit 1 bis 4 Kohlenstoff-Atomen, das gegebenenfalls durch Halogen substituiert ist, oder eine Alkylsulfonyloxy-Gruppe mit 1 bis 4 Kohlenstoff-Atomen, die gegebenenfalls durch Halogen substituiert ist, bezeichnet,

X CH oder N bezeichnet und

$R^1$, $R^2$ und $R^3$ jeweils Wasserstoff oder eine Alkyl-Gruppe mit 1 bis 4 Kohlenstoff-Atomen, die gegebenenfalls durch Halogen substituiert ist, bezeichnen, mit der Maßgabe, daß nicht $R^1$, $R^2$ und $R^3$ gleichzeitig Wasserstoff bezeichnen; oder $R^2$ und $R^3$ zusammen mit dem Kohlenstoff-Atom, an das sie gebunden sind, einem alicyclischen Ring mit 3 bis 6 Kohlenstoff-Atomen bilden können,

dadurch gekennzeichnet, daß

a) in dem Fall, in dem A Hydroxy bezeichnet, Verbindungen der Formel (II)

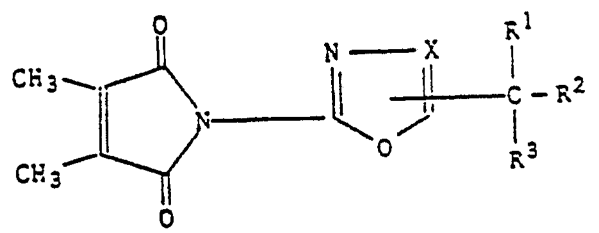

(II)

,

in der X, $R^1$, $R^2$ und $R^3$ jeweils die im Vorstehenden angegebenen Bedeutungen haben, mit einem Reduktionsmittel in Gegenwart inerter Lösungsmittel umgesetzt werden oder

b) in dem Fall, in dem A Halogen bezeichnet, Verbindungen der Formel (Ia)

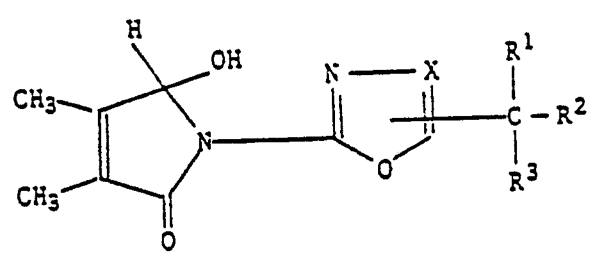

(Ia)

,

in der X, $R^1$, $R^2$ und $R^3$ jeweils die im Vorstehenden angegebenen Bedeutungen haben, mit einem Halogenierungsmittel in Gegenwart inerter Lösungsmittel umgesetzt werden oder

c) in dem Fall, in dem A eine Alkylcarbonyloxy-Gruppe mit einem Alkyl mit 1 bis 4 Kohlenstoff-Atomen, das gegebenenfalls durch Halogen substituiert ist, oder eine Alkylsulfonyloxy-Gruppe mit 1 bis 4 Kohlenstoff-Atomen, die gegebenenfalls durch Halgoen substituiert ist, bezeichnet, A durch -O-W etsetzt wird,

die Verbindungen der oben bezeichneten Formel (Ia) mit Verbindungen der Formel (III)

W - M (III),

worin

W eine Alkylcarbonyl-Gruppe mit einem Alkyl mit 1 bis 4 Kohlenstoff-Atomen, das gegebenenfalls durch Halogen substituiert ist, oder eine Alkylsulfonyl-Gruppe mit 1 bis 4 Kohlenstoff-Atomen, die gegebenenfalls

durch Halogen substituiert ist, gezeichnet und

M Halogen bezeichnet, oder

mit Verbindungen der Formel (IV)

W - O - W (IV),

worin W die im Vorstehenden angegebenen Bedeutungen hat,

in Gegenwart inerter Lösungsmittel und gegebenenfalls in Gegenwart einer Base umgestzt werden.

6. Herbizide Mittel, dadurch gekennzeichnet, daß sie wenigstens eine Verbindung der Formel (I) nach den Ansprüchen 1 bis 5 enthalten.

7. Verfahren zur Bekämpfung von Unkräutern, dadurch gekennzeichnet, daß Verbindung der Formel (I) nach den Ansprüchen 1 bis 5 auf Unkräuter und/oder deren Lebensraum zur Einwirkung gebracht werden.

8. Verwendung von Verbindungen der Formel (I) nach den Ansprüchen 1 bis 5 zur Bekämpfung von Unkräutern.

9. Verfahren zur Herstellung herbizider Mittel, dadurch gekennzeichnet, daß Verbindungen der Formel (I) nach den Ansprüchen 1 bis 5 mit Streckmitteln und/oder grenzflächenaktiven Mitteln vermischt werden.

10. Verbindungen der Formel (II)

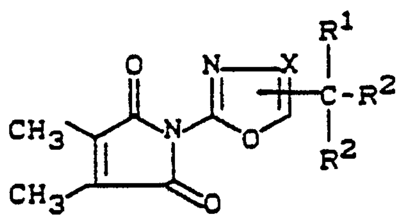

(II)

,

in der

X CH oder N bezeichnet und

$R^1$, $R^2$ und $R^3$ jeweils Wasserstoff oder eine Alkyl-Gruppe mit 1 bis 4 Kohlenstoff-Atomen, die gegebenenfalls durch Halogen substituiert ist, bezeichnen, mit der Maßgabe, daß $R^1$, $R^2$ und $R^3$ nicht gleichzeitig Wasserstoff bezeichnen, oder $R^2$ und $R^3$ zusammen mit dem Kohlenstoff-Atom, an das sie gebunden sind, einen alicyclischen Ring mit 3 bis 6 Kohlenstoff-Atomen bilden können.

Europäisches Patentamt

# EUROPÄISCHER RECHERCHENBERICHT

Nummer der Anmeldung

EP 89106689.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CH - A5 - 633 678 (CIBA-GEIGY) * Verbindungen I,IV,27; Seite 2, Spalte 2, Zeile 67 - Seite 3, Spalte 1, Zeile 9 * -- | 1,6,10 | C 07 D 413/04<br>A 01 N 43/76<br>A 01 N 43/82 |
| A | FR - A1 - 2 400 013 (CIBA-GEIGY) * Verbindungen I,IV * -- | 1,10 | |
| D,A | DE - A1 - 2 735 841 (CIBA-GEIGY) * Ansprüche 1,12; Verbindung II * ---- | 1,6,10 | |
| | | | RECHERCHIERTE SACHGEBIETE (Int. Cl.4) |
| | | | C 07 D 413/00 |

Der vorliegende Recherchenbericht wurde für alle Patentansprüche erstellt.

| Recherchenort | Abschlußdatum der Recherche | Prüfer |
|---|---|---|
| WIEN | 01-08-1989 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument